# FASCICULE DE BREVET EUROPEEN

(11) **EP 0 376 908 B1**
(45) Date de publication et mention de la délivrance du brevet: **27.01.1993**
(21) Numéro de dépôt: 89870173.5
(22) Date de dépôt: 06.11.1989
(51) Int. Cl.: G01N 27/49, G01N 27/48, G01N 33/18

(54) **Procédé de mesure en continu de cyanure dans les eaux résiduaires de la sidérurgie et appareillage à cet effet**
Verfahren und Vorrichtung zur kontinuierlichen Messung von Cyanid in Abwässern der Eisenindustrie
Method for continuously measuring cyanide in waste waters from steel making and apparatus therefor

(30) Priorité: 30.12.1988 BE 8801455
(43) Date de publication de la demande: 04.07.1990
(73) Titulaire: COCKERILL SAMBRE Société Anonyme dite:, B-4100 Liege (Seraing) (BE)
(72) Inventeur: Propson, Raymond Emile Hubert, B-4910 Liège (Angleur) (BE); Hissel, Joseph Alfred, B-4801 Stembert (BE)
(74) Mandataire: Bosch, Henry

(56) Documents cités:
- US-A- 4 227 888
- FRESENIUS ZEITSCHRIFT FUER ANALYTISCHE CHEMIE, vol. 286,no. 5,20 octobre 1977, pages 351-354, Springer-Verlag, Berlin, DE; K. WISSER: "Spurenbestimmung von Cyanid mittels polarographischer Differential-Puls-Technik"
- COLLECTION OF CZECHOSLOVAK CHEMICAL COMMUNICATION, vol. 47, 1982, pages 2214-2218, Prague, CS; V. STARA et al.: "Fast scan differential pulse polarographic determination of cyanide"
- ANALYTICA CHIMICA ACTA, vol. 131, 1981, pages 167-174, Amsterdam, NL; L.K. LEUNG et al.: " Normal pulse polarographic quantification of cyanide and sulfide by the anodization of mercury"

## Description

### Objet de l'invention

L'invention s'adresse à une technique de mesure en continu de l'ion cyanure dans les eaux résiduaires des installations sidérurgiques. Elle s'étend à un appareillage convenant pour une telle mesure.

### But visé par l'invention

Certaines branches de l'industrie sidérurgique au sens large peuvent être la cause de pollutions des eaux résiduaires par suite de la libération dans celles-ci de cyanure. Les installations de cokerie et de haut-fourneau notamment peuvent être la cause de la présence de cyanure dans les eaux de rejet industrielles. La cause précise de la formation de cyanures et de la façon de les éviter sont mal connues, ce qui peut provoquer des accroissements incontrôlables et brutaux de la teneur en cyanure dans les eaux de rejet.

La présence du cyanure dans les eaux de rejet industrielles constitue un danger très sérieux pour la vie aquatique des cours d'eau récepteurs. De plus, certaines eaux de surface étant utilisées après traitement pour la consommation humaine, leur pollution doit être aussi réduite que possible.

C'est pourquoi les autorités compétentes en matière de pollution ont fixé la norme de rejet (rivière) à 1 mg/l. On comprend dès lors qu'il soit impératif de procéder à un contrôle constant de la concentration en cyanure dans les effluents.

Par ailleurs, il est bien connu que les teneurs en cyanure dans les eaux de rejets sont sujettes à des fluctuations importantes dues notamment à des variations difficilement contrôlables du taux de production ou de la marche des engins. Il s'en suit qu'il y a lieu de réduire au strict minimum le temps nécessaire à l'analyse de façon à prévenir tout rejet dangereux en cas d'augmentation rapide de la concentration en cyanure, pour pouvoir prendre immédiatement les mesures de protection qui s'imposent.

Une méthode de mesure pratiquement instantanée et en continu du cyanure est donc très souhaitable.

### Etat de la technique

Les cyanures sont le plus souvent accompagnés dans les eaux résiduaires de diverses substances qui interfèrent dans le dosage. C'est pourquoi, il est le plus souvent nécessaire de prévoir une étape de séparation qui consiste habituellement en une distillation, dans laquelle on utilise la propriété de l'acide cyanhydrique de pouvoir se volatiliser et être ainsi isolé de la majorité des substances gênantes.

Cette opération de distillation rend impossible une mesure en continu par suite de sa durée de l'ordre de 75 à 130 minutes.

Dans le cas des eaux résiduaires de la sidérurgie, on n'a pas à tenir compte de la présence des complexes que forme l'ion CN⁻ avec la majorité des métaux lourds. Les seuls complexes existants sont ceux du fer (ferrocyanure et ferricyanure) qui ne sont pas toxiques. Cette spécificité permet d'éviter les distillations généralement pratiquées.

Des techniques de mesure polarographiques ont été décrites qui visent à déterminer la concentration en cyanure libre en présence de différentes substances qui interfèrent dans les autres types de détermination. En particulier, les interférences dues aux sulfures, sulfites, sulfocyanures, cyanates, chlorures sont citées dans les publications ci-après comme étant sans influence sur la mesure de la concentration en cyanure, dans le cas de la détermination polarographique:
- D.R. CANTERFORD
   Analyt. Chem. 1975, 47, 88-92
- E. KIROWA-EISNER, D. TALMOR et J. OSTERYOUNG
   Analyt. Chem. 1981, 52, 581583
- L.K. LEUNG et D.E. BARTAK
   Analytica Chimica Acta, 1981, 131, 167-174
- METROHM APPLICATION - Bulletin, n° 110 f
- K. WISSER
   Z. Anal. Chem. 1977, 286, 351-354.

Ces procédés diffèrent par leur mode opératoire; par ailleurs, ils ne semblent pas avoir été conçus pour tenir compte de la spécificité des problèmes rencontrés en sidérurgie, en particulier pour le dosage des cyanures dans les eaux de hauts-fourneaux et de cokeries.

En pratique on observe la présence dans les eaux résiduaires de la sidérurgie, et en particulier dans les eaux de cokerie une quantité importante d'ions S₂O₃⁻⁻ (thiosulfate). Bien que les pics CN⁻ et S₂O₃⁻⁻ apparaissent à des potentiels assez différents (CN⁻ = ^{∼} - 300 mV (Vs Ag/AgCl) et (S₂O₃⁻⁻ = ^{∼} - 150 mV), le très important excès de S₂O₃⁻⁻ fait en sorte que les 2 pics se recouvrent partiellement.

En effet, la largeur d'un pic est d'autant plus importante que sa concentration est grande, ce qui est le cas pour l'ion S₂O₃⁻⁻. Ceci pose un problème pour l'évaluation quantitative de cyanure dans de telles eaux.

On constate dans les mesures que le cyanure est partiellement masqué par la présence du fond continu dû au thiosulfate S₂O₃⁻, ce qui rend malaisée l'évaluation quantitative.

L'invention permet de résoudre cette difficulté.

### Eléments caractéristiques de l'invention

L'invention concerne un procédé de mesure polarographique consistant à procéder à deux mesures successives, à savoir:
- mesure de la somme des courants dus à CN⁻ et S₂O₃⁻⁻ au potentiel correspondant à la valeur maximale du pic CN⁻;
- mesure du pic dû au seul ion S₂O₃⁻⁻ à ce même potentiel, après avoir éliminé les cyanures par l'action du formaldéhyde en formant une cyanhydrine, qui ne donne pas de signal polarographique,

la différence entre les deux courbes obtenues permettant d'obtenir une courbe résultante corrigée de toute interférence gênante.

Selon une forme d'exécution préférée de l'invention, on utilise pour ce type de mesure une méthode polarographique moderne, à savoir la polarographie impulsionnelle différentielle.

Le signal obtenu par cette méthode (intensité de courant en fonction du potentiel) consiste en une courbe prenant l'allure d'un pic.

Il y a lieu de retenir que la hauteur du pic est proportionnelle à la concentration en cyanure libre, ce qui facilite son exploitation.

La technique de l'invention permet en conséquence de mesurer les cyanures dans les eaux résiduaires sidérurgiques telles que les eaux de cokerie et de hauts-fourneaux, sans séparation préalable.

En faisant appel à cette méthode, on arrive à éliminer pratiquement l'action perturbatrice de presque toutes les substances de nature à interférer sur la mesure, même lorsque le rapport des concentrations [S₂O₃⁻⁻]/[CN⁻] est élevée comme c'est souvent le cas pour les applications spécifiques envisagées selon l'invention.

La technique par automatisation des opérations consécutives du dosage permet une analyse avec une périodicité pouvant atteindre environ 10 mesures par heure.

D'autres caractéristiques inventives apparaîtront à la lecture de la description d'un mode d'exécution préféré qui suit.

### Description détaillée de l'invention

Le principe de la mesure polarographique repose sur l'oxydation anodique du mercure à l'électrode à goutte tombante.

En présence d'ions cyanure, il se forme des complexes mercurocyanurés Hg(CN)₂, Hg(CN)₃⁻, Hg(CN)₄⁻⁻.

Le mécanisme de réaction est sous contrôle de la vitesse de diffusion de l'ion CN⁻, laquelle est proportionnelle à la concentration en cyanure (CN)⁻.

Il en résulte que la mesure polarographique permet d'atteindre directement la concentration en cyanure libre.

Comme la mesure est effectuée en milieu suffisamment alcalin, tout l'acide cyanhydrique HCN est présent sous forme de CN⁻.

De sorte que la concentration en cyanure libre CN⁻ représente la fraction des cyanures non liés à un métal sous forme de complexe métallocyanure.

Un appareillage convenant pour la mise en oeuvre de la technique de l'invention peut être constitué par un dispositif de prélèvement direct ou par partie aliquote d'une quantité déterminée d'échantillons qui est alimenté vers un polarographe relié à un processeur de mesure qui relève en continu la courbe polarographique.

L'échantillon après mesure est traité par une quantité suffisante de formaldéhyde pour détruire la totalité des ions cyanure après quoi l'échantillon traité subit une deuxième mesure.

Les résultats enregistrés pour les deux mesures successives sont traités dans une unité qui calcule la différence entre les deux courbes relevées successivement et intègre les résultats ainsi obtenus en fournissant la concentration en cyanure.

Ces résultats peuvent être affichés, imprimés, ou peuvent servir à déclencher en cas de dépassement d'une valeur déterminée une alarme ou une commande interrompant automatiquement le rejet des eaux résiduaires en le dirigeant vers une installation de traitement adéquate.

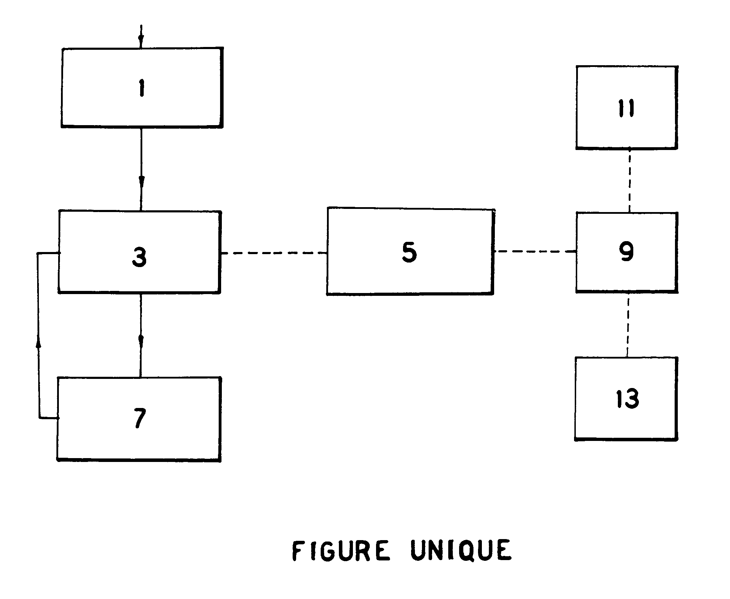

### Mode d'exécution illustré par le dessin annexé

Le dessin annexé représente schématiquement le principe de fonctionnement d'un appareillage pour la mise en oeuvre de l'invention, sous forme de blocs fonctionnels.

Les traits continus représentent une circulation d'un échantillon, les traits discontinus la transmission de données.

Le bloc 1 représente l'opération de prélèvement d'un échantillon pour la mesure. Cet échantillon peut être prélevé automatiquement à des cadences régulières (par exemple dix prélèvements par heure) dans les effluents d'une industrie sidérurgique.

L'échantillon peut subir, si nécessaire, une préparation adéquate, telle qu'une filtration.

L'échantillon est alimenté vers un polarographe illustré par le repère 3 pour une première mesure, les résultats étant transmis vers le processeur 5 qui mémorise la valeur mesurée.

A la fin de la mesure, l'échantillon est traité en 7 par un excès de formaldéhyde en formant une cyanhydrine et il est ensuite soumis à une deuxième mesure dont le résultat est traité par le processeur 5. Ce processeur compare les résultats de la première et de la deuxième mesure pour en tirer la valeur résultant de la présence de cyanure selon les principes à la base de l'invention.

Le processeur 5 peut être relié à un terminal 9 (PC) permettant d'enregistrer en continu les valeurs obtenues.

Ce terminal 9 peut à son tour commander tout dispositif adéquat tel qu'une imprimante 11 et/ou une installation d'alarme 13 qui se met en fonctionnement lorsqu'une valeur de consigne déterminée est dépassée.

Bien entendu, un tel appareillage doit être équipé de moyens classiques pour assurer la mesure tels que des moyens de pipettage, des moyens de dilution et des moyens de pompage pour assurer la circulation des échantillons.

## Revendications

1. Procédé de mesure en continu de cyanure dans les eaux résiduaires de la sidérurgie par polarographie caractérisé en ce qu'on procède à deux mesures successives, à savoir:
- mesure de la somme des courants dus à CN⁻ et S₂O₃⁻⁻ au potentiel correspondant à la valeur maximale du pic CN⁻;
- mesure du pic dû au seul ion S₂O₃⁻⁻ à ce même potentiel, après avoir éliminé les cyanures par l'action du formaldéhyde en formant une cyanhydrine, qui ne donne pas de signal polarographique,
la différence entre les deux courbes obtenues permettant d'obtenir une courbe résultante corrigée de toute interférence gênante.

2. Procédé selon la revendication 1 caractérisé en ce qu'on a recours à la méthode de polarographie impulsionnelle différentielle.

3. Procédé selon la revendication 1 ou 2 caractérisé en ce qu'il est automatisé pour permettre des analyses avec une périodicité pouvant atteindre environ 10 mesures par heure.

4. Procédé selon l'une quelconque des revendications 1 caractérisé en ce qu'il est effectué sur des échantillons en l'absence d'une étape de distillation préalable.

## Patentansprüche

1. Verfahren zur kontinuierlichen Messung von Cyanid in Abwässern der Eisenhüttenindustrie mittels Polarographie, dadurch gekennzeichnet, daß in zwei aufeinanderfolgenden Messungen verfahren wird, nämlich:
- Messung der Spitze der Ströme entsprechend CN- und S₂O₃-- an dem Potential, das einem Maximalwert der CN-Spitze entspricht;
- Messung der Spitze entsprechend dem einzigen Ion S₂O₃-- an diesem Potential, nachdem das Cyanid durch die Aktivität des Formaldehyds unter Bildung eines Cyanhydrins eliminiert worden ist, welcher kein polarographisches Signal abgibt,
wobei die Differenz zwischen den beiden erhaltenen Kurven es ermöglicht, eine daraus resultierende Kurve zu erstellen, aus der alle störenden Interferenzen herauskorrigiert sind.

2. Verfahren gemäß Anspruch 1, dadurch gekennzeichnet, daß das differentielle Puls-Polarographie-Verfahren angewendet wird.

3. Verfahren gemäß Anspruch 1 oder 2, dadurch gekennzeichnet, daß es automatisiert ist, derart, daß Analysen mit einer Regelmäßigkeit ausgeführt werden können, die bis zu etwa zehn Messungen pro Stunde gehen kann.

4. Verfahren gemäß einem der Ansprüche 1 bis 3, dadurch gekennzeichnet, daß es unter Abwesenheit eines vorangehenden Destillationsschrittes an den Proben ausgeführt wird.

## Claims

1. A method of continuously measuring the quantity of cyanide in iron and steel works process water by polarography, characterised in that two successive measurements are made to establish:
- the value of the sum of the current due to CN⁻ and S₂O₃⁻⁻ at the voltage corresponding to the maximum value of the CN⁻ peak;
- the value of the peak due solely to the S₂O₃⁻⁻ at this same voltage, having eliminated the cyanides by the action of formaldehyde, forming a cyanhydrin which gives no polarograph signal,
the difference between the two curves thus obtained allowing a resultant curve to be obtained corrected for all disturbing interference.

2. A method according to claim 1, characterised in that the method of differential impulse polarography is used.

3. A method according to claim 1 or 2, characterised in that the method is automated to allow up to about ten measurements per hour.

4. A method according to any preceding claim, characterised in that the analysis is carried out on samples without a previous distillation stage.
